(19) Europäisches Patentamt
European Patent Office
Office européen des brevets

(11) **EP 2 163 201 A1**

(12) **EUROPEAN PATENT APPLICATION**

(43) Date of publication:
**17.03.2010 Bulletin 2010/11**

(51) Int Cl.:
***A61B 6/03*** *(2006.01)*

(21) Application number: **08105352.2**

(22) Date of filing: **15.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
• **Westfälische Wilhelms-Universität Münster**
  **48149 Münster (DE)**
• **Siemens Medical Solutions USA, Inc.**
  **Malvern, PA 19355-1406 (US)**

(72) Inventors:
• **Büther, Florian**
  **48151 Münster (DE)**

• **Schäfers, Klaus**
  **48291 Telgte (DE)**
• **Jones, William F.**
  **Knoxville, TN 37932 (US)**
• **Bendriem, Bernard**
  **Knoxville, TN 37919 (US)**
• **Newiger, Hartwig**
  **90427 Nürnberg (DE)**

(74) Representative: **Molnia, David et al**
  **df-mp**
  **Fünf Höfe**
  **Theatinerstrasse 16**
  **80333 München (DE)**

(54) **List mode-based respiratory and cardiac gating in positron emission tomography**

(57)    According to a preferred embodiment, the invention provides a method for extracting internal organ motion from positron emission tomography (PET) coincidence data, the method comprising the following steps: generating a data stream of PET coincidence data using the list mode capability of a PET scanner; dividing the data stream into time frames of a given length; computing a histogram $A(i, t)$ of an axial coincidence distribution for a set of time frames; computing the axial center of mass $z(t)$ for each of the time frames in the set of time frames based on the histogram $A(i, t)$; transforming $z(t)$ into the frequency domain; determining either the frequency contribution caused by respiratory motion, given by $f_{resp}$, or the frequency contribution caused by heart contractions, given by $f_{card}$ and $\Delta f$, identified in the frequency spectrum $|Z(f)|$; and carrying out further processing of $Z(f)$ leading to curves $z_{resp}(t)$ and $z_{card}(t)$ with which a gating sequence is established.

Fig.1

EP 2 163 201 A1

## Description

Background of the invention

Technical field

The invention relates to a method for extracting internal organ motion directly from PET coincidence data. More particularly, the invention relates to a method for extracting internal organ motion directly from PET coincidence data in the case of myocardial viability FDG scans.

Description of the prior art

The combination of Positron emission tomography (PET) and computed tomography (CT) has proven to be a valuable tool in medical imaging, in particular because each of these modalities gathers different information that when combined improve medical diagnosis.

PET is based on positron-emitting isotopes like $^{18}$F which are attached to specific molecules (called tracers; e.g., FDG) and which are introduced into the human body. Emitted positrons annihilate with an electron close to its origin of emission, resulting in two gamma photons of 511 keV that can be detected outside the body. The detection of such photons may be used to create images comprising functional or metabolic information. CT is based on x-ray transmission measurements through the human body, therefore resulting in morphological tissue density images.

One problem in PET is the fact that not all generated gamma photons can be detected as many photons travelling through tissue undergo absorption processes. This problem is in principle resolvable (attenuation correction) as long as the (gamma) density of the human body inside the field of view is known. In PET/CT, this information is gathered using the acquired CT data, usually resulting in fast and reliable PET attenuation correction. However, cardiovascular PET/CT faces further difficulties based on the difference in scanning time between CT (usually a few seconds) and PET (a few minutes per bed position).

Thus CT images only represent one respiratory phase, while PET images comprise a superposition of all phases during the PET scan, leading to image blurring, therefore effectively reducing the image resolution. Furthermore, CT-based attenuation correction may introduce image artifacts in such cases where the CT respiratory phase does not match the averaged PET respiratory phase. The result may be a spatial mismatch between CT and PET and erroneous tracer quantification (Lang N, Dawood M, Büther F, Schober O, Schäfers M, Schäfers K. Organ Movement-Reduction in PET/CT using Dual-Gated List mode Acquisition. Z Med Phys. 2006; 16:93-100).

Different strategies to overcome these problems have been proposed; e.g., usage of a "slow CT" (Souvatzaglou M, Bengel F, Busch R, et al. Attenuation correction in cardiac PET/CT with three different CT protocols: a comparison with conventional PET. Eur J Nucl Med Mol Imaging 2007;34:1991-2000) which comprises some respiratory cycles, aiming to simulate motion-blurred CT data corresponding to the PET data. However, it is not clear if a few breathing cycles really match the situation during the PET scan. Moreover, this does not solve the problem of motion blurring in the obtained images.

One well-known method for obtaining both blurring- and attenuation artifact-free images is the method of gating. In such a method, the acquired PET data is retrospectively divided into subsets referred to as gates according to a breathing curve that is recorded during the PET scan. These gates may then be reconstructed independently, resulting in PET images representing only one respiratory phase without motion-based blurring. If only the PET gate representing the CT respiratory phase is used for reconstruction, the attenuation-corrected PET image is devoid of attenuation correction artifacts.

A crucial point of this approach is the acquisition of the breathing motion during the PET scan which can be achieved by different methods. The literature suggests the application of a pressure-sensitive sensor that measures the respiration-induced pressure changes on the patient's abdomen during the PET scan (Klein GJ, Reutter BW, Ho MW, Reed JH, Huesman RH. Real-time system for respiratory-cardiac gating in positron tomography. IEEE Trans Nucl Sci. 1998;45:2139-2143), usage of an infrared tracking device computing the position of markers placed on the abdomen (Nehmeh SA, Erdi YE, Ling CC, et al. Effects of Respiratory Gating on Reducing Lung Motion Artifacts in PET Imaging of Lung Cancer. Med Phys. 2002;29(3):366-371), and usage of a video camera placed at the end of the patient bed, monitoring the respiratory motion of the patient (Dawood M, Büther F, Lang N, Schober O, Schäfers KP. Respiratory gating in positron emission tomography: a comparison of different gating schemes. Med Phys. 2007;34(7):3067-3076). Further suggestions include temperature sensors that measure the flow of the respiration air (Boucher L, Rodrigue S, Lecomte R, Bernard F. Respiratory gating for 3-dimensional PET of the thorax: Feasibility and initial results. J Nucl Med. 2004;45:214-2I9) or usage of radioactive sources inside the PET field of view placed on the patient as external motion marker (Nehmeh SA, Erdi YE, Rosenzweig KE, et. al. Reduction of respiratory motion artifacts in PET imaging of lung cancer by respiratory correlated dynamic PET: methodology and comparison with respiratory gated PET. J Nucl Med. 2003 ;44: 1644-1648).

A disadvantage of all these methods is the usage of additional hardware during the PET/CT scan, introducing additional potential errors of measurement. Beyond that, only external motion parameters are measured which may not be well correlated to internal heart motion.

As a matter of fact, a clinical study using magnetic resonance scans proved a certain, yet not perfect correlation between external and internal motion (Koch N, Liu HH, Starkschall G, et al. Evaluation of internal lung motion for respiratory-gated radiotherapy using MRI: Part I-correlating internal lung motion with skin fiducial motion, Int J Radiat Oncol Biol Phys. 2004;60(5): 1459-1472).

Thus, a gating method that incorporates internal motion information into the gating process with as little effort in soft- and hardware as possible would be desirable. Such approaches are already under development in the field of oncological PET/CT; here, the respiratory signal is extracted from the PET list mode data itself (Bundschuh R A, Martinez-Moeller A, Essler M, et al. Postacquisition Detection of Tumor Motion in the Lung and Upper Abdomen Using list mode PET Data: A Feasibility Study. J Nud Med. 2007;48:758-763). Unfortunately, these methods are extraordinarily time-consuming (as each time frame has to be reconstructed before extracting motion information) and therefore much too intricate for clinical studies.

It is the object of the present invention to allow extracting internal motion information of the heart directly from the PET data itself. This is done without additional hardware; additionally, it is very time-efficient and superior to respiratory gating methods that rely on external motion information.

### Summary of the invention

The invention provides an improved method for extracting internal organ motion directly from PET coincidence data. More particularly, the invention provides a method for extracting internal organ motion directly from PET coincidence data in the case of myocardial viability FDG scans.

According to a preferred embodiment of the invention, the method comprises the following steps: generating a data stream of PET coincidence data using the list mode capability of a PET scanner; dividing the data stream into time frames of a given length; computing a histogram $A(i, t)$ of the axial coincidence distribution for a set of time frames; computing the axial center of mass $z(t)$ for each of the time frames in the set of time frames based on the histogram $A(i, t)$; transforming $z(t)$ into the frequency domain; determining either the frequency contribution caused by respiratory motion, given by $f_{resp}$, or the frequency contribution caused by heart contractions, given by $f_{card}$ and $\Delta f$, identified in the frequency spectrum $|Z(f)|$; and carrying out further processing of $Z(f)$ leading to a curve $z_{resp}(t)$ or $z_{card}(t)$ with which a gating sequence is established.

The list mode data stream comprises coordinates of measured PET coincidences. The further processing of $Z(f)$ comprises carrying out an inverse Fourier transformation (iFFT). $Z(f)$ comprises $Z_{resp}$, the spectrum of respiratory frequencies and $Z_{card}$, the spectrum of heart contraction frequencies. $z(t)$ comprises the res-

piratory curve $z_{resp}(t)$ and the cardiac curve $z_{card}(t)$. The list mode data stream comprises time tags. The length of the time frames can be set to be in a range from 5ms to 200ms, wherein the preferred length of a time frame is 50ms. Computing the axial coincidence distribution requires the extraction of the axial coordinate for every coincidence from the list mode data. In case of coincidences belonging to higher segments of the michelogram, a single slice rebinning is performed. With single slice rebinning, prompt and delayed coincidences are taken into account with positive and negative weight, respectively. Using a fast fourier transformation (FFT), the axial center of mass $z(t)$ is transformed into the frequency domain. The values for $f_{resp}$, $f_{card}$ and $\Delta f$ are found either manually or, by smoothing the spectrum, automatically.

According to another embodiment, the invention provides a method for extracting internal organ motion from positron emission tomography (PET) coincidence data, the method comprising the following steps: generating a data stream of PET coincidence data using the list mode capability of a PET scanner; dividing the data stream into time frames of a given length; computing a histogram $A(i, t)$ of an axial coincidence distribution for a set of time frames; computing the axial center of mass $z(t)$ for each of the time frames in the set of time frames based on the histogram $A(i, t)$; and applying a Savitzky Golay filter to the raw curve $z(t)$ leading to a respiratory signal with which a gating sequence is established.

According to yet another embodiment, the invention provides a method for extracting internal organ motion from positron emission tomography (PET) coincidence data, the method comprising the following steps: generating a data stream of PET coincidence data using the list mode capability of a PET scanner; dividing the data stream into time frames of a given length; computing a histogram $A(i, t)$ of the axial coincidence distribution for a set of time frames; computing the distribution's standard deviation $\Delta z(t)$ based on the histogram $A(i, t)$; transforming $\Delta z(t)$ into the frequency domain; determining either the frequency contribution caused by respiratory motion, given by $f_{resp}$, or the frequency contribution caused by heart contractions, given by $f_{card}$ and $\Delta f$, identified in the frequency spectrum $|\Delta Z(f)|$; and carrying out further processing of $\Delta Z(f)$ leading to curves $\Delta z_{resp}(t)$ and $\Delta z_{card}(t)$ with which a gating sequence is established.

### Brief description of the drawings

Fig. 1A is a schematic diagram of a preferred embodiment of the present invention showing the list mode data stream being divided into frames.

Fig. 1B is a schematic diagram of a preferred embodiment of the present invention showing that in each frame, a histogram of the axial coincidence distribution being computed.

Fig. 1C is a schematic diagram of a preferred embodiment of the present invention showing the axial center of mass z(t) for each frame being computed.

Fig. 2 shows an example of a computed center of mass-curve z(t) on the left and its spectrum |Z(f)| on the right according to a preferred embodiment of the present invention.

Fig. 3 shows the isolated respiratory part of the spectrum $|Z_{resp}(f)|$ on the left and the corresponding computed respiratory curve $z_{resp}(t)$ on the right according to a preferred embodiment of the present invention.

Fig. 4 shows the isolated cardiac part of the spectrum $|Z_{card}(f)|$ on the left and the corresponding computed respiratory curve $z_{card}(t)$ on the right according to a preferred embodiment of the present invention.

Fig. 5 shows on the left: PET image without gating, in the middle: PET image in maximum expiration; on the right PET image in maximum inspiration according to a preferred embodiment of the present invention.

Fig. 6 shows on the left: PET image without gating; in the middle: PET image in end systole; on the right: PET in end diastole according to a preferred embodiment of the present invention.

Detailed description of the invention

The present invention is based on extracting internal organ motion from PET coincidence data. The embodiments of the present invention described hereinafter require using a PET scanner with list mode capability.

According to a preferred embodiment of the present invention, the case where heart contraction is connected to an axial motion shift and a heart beat peak is visible in the spectrum is described. The axial center of mass is then plotted as a function of time. Using a Fourier transform, the data is transformed into the frequency domain making it possible to identify and in turn isolate the respiratory and cardiac part of the spectrum respectively. Using an inverse Fourier transform, respiratory and cardiac curves can be computed with which a gating sequence can then be established.

According to another embodiment of the present invention, the case where heart contraction is not connected to an axial motion shift and no heart beat peak is visible in the spectrum is described. This requires an alternate approach. Instead of computing the axial center of mass, a computation of the distribution's standard deviation reveals a signal of the heart beat which can then be plotted against time and transformed into the frequency domain allowing isolation of the respiratory and cardiac part of the spectrum respectively. Like in the preferred embodiment, using an inverse Fourier transform, respiratory and cardiac curves can then be computed with which a gating sequence can subsequently be established.

According to yet another embodiment of the present invention, the case is described where instead of using a Fourier analysis, a Savitzky Golay filter is applied to the raw curve suppressing higher frequencies and resulting in a respiratory signal with which a gating sequence can then be established.

In the following the preferred embodiment of the present invention is described in more detail. It is especially valuable in the case of cardiac viability studies using FDG, as most emitted photons have their origin in the usually high tracer concentrations in the myocardium.

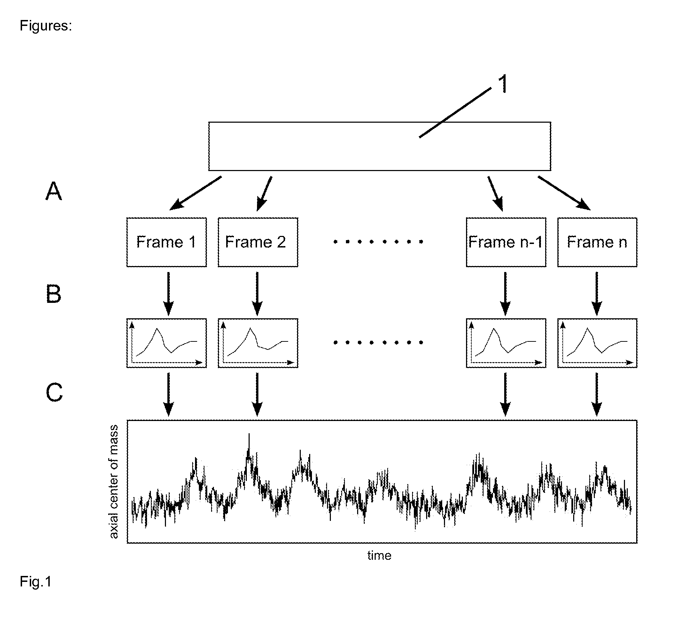

Fig. 1 illustrates a basic scheme of the proposed list mode gating. In a first step, a list mode data stream (1) is divided into a set number of time frames of a given length. In particular, the set of time frames can comprise the entire data stream or can comprise only a part of said data stream. The list mode data stream (1) comprises the coordinates of measured PET coincidences (both prompt and delayed) in addition to time tags. The length of the time frames can be set to be in a range from 5ms to 200ms, wherein the preferred length of a time frame is 50ms (Fig. 1A). The set of time frames taken from the list mode data stream (a primary set of time frames) can further be subdivided into a smaller set of time frames (a secondary set of time frames), e.g. by selecting every second or $n^{th}$ time frame of the primary set of time frames.

For the primary or - if available - secondary set of time frames a histogram of the axial coincidence distribution is computed for each time frame of the set of time frames (Fig. 1B). This requires the extraction of the axial coordinate of each coincidence (= slice number) from the list mode data (1). In case of coincidences belonging to a higher segment of the michelogram (coincidences between two distinct detector rings), a single slice rebinning (SSRB) is performed. Prompt and delayed coincidences are taken into account with positive and negative weight, respectively. So for each frame a histogram A(i, t) can be derived (i being the slice number, t being the frame number). This is further processed by computing the axial center of mass z(t) for each frame according to

$$z(t) = \frac{\sum_i i \cdot A(i,t)}{\sum_i A(i,t)}$$

(Fig. 1C).

Hence, heart contractions connected to an axial motion shift are made visible in a chart of the axial center of mass versus time.

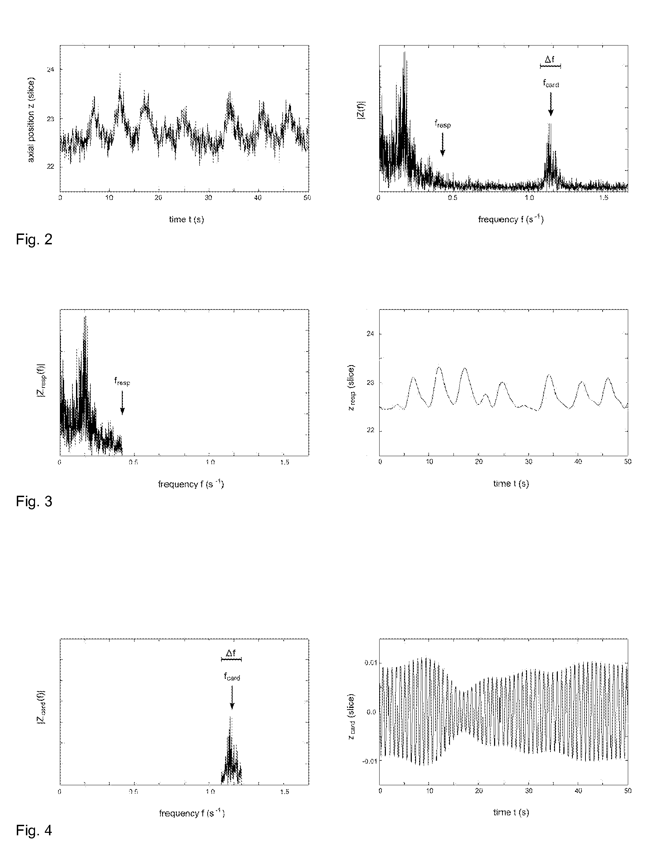

This results in a curve of the axial center of mass as a function of scanning time. Fig. 2 is an example of a computed center of mass curve z(t) on the left (detail of a 20 minute FDG PET scan) and its spectrum |Z(f)| on the right. The low frequency parts up to $f_{resp} \approx 0.5$ Hz represent the respiratory motion; the peak at $f_{card} \approx 1.1$ Hz represents cardiac contractions.

It is clear that z(t) will change according to a (more or less) uniform motion (respiratory motion, heart contraction) of tracer concentrations along the scanner's axis present during the scan, however, the curve is also affected by the statistical nature of radioactive decay, resulting in a certain amount of noise in z(t). Using a discrete fast fourier transformation (FFT), z(t) is transformed into the frequency domain:

$$Z(f) = FFT[z(t)]$$

Typically, three components can be identified in the frequency spectrum |Z(f)|:

- A background evenly distributed over the whole frequency range, caused by the aforementioned statistical nature of decay;
- A low frequency contribution caused by respiratory motion and usually limited to values lower than $f_{resp} \approx 0.5$ Hz.
- A contribution caused by heart contractions centered around a frequency $f_{card} \approx 1$ Hz with a width of $\Delta f \approx 0.15$ Hz.

The values for $f_{resp}$, $f_{card}$ and $\Delta f$ can be found either manually or, by smoothing the spectrum, automatically.

Respiratory motion can now be separated by confining the spectrum to respiratory frequencies up to $f_{resp}$:

$$Z_{resp}(|f| < f_{resp}) = Z(f)$$

$$Z_{resp}(|f| > f_{resp}) = 0$$

An inverse Fourier transformation iFFT of Z(f) finally leads to the respiratory curve $z_{resp}$ (t):

$$Z_{resp}(t) = iFFT[Z_{resp}(f)]$$

with which a gating sequence can easily be established (Fig. 3). Possible gating schemes are e.g. equal and variable time-based gating which uses only the time information of the breathing cycle to define respiratory gates, or equal and variable amplitude-based gating which utilizes the amplitude of the respiratory signal.

Fig. 3 shows the isolated respiratory part of the spectrum |$Z_{resp}$ (f)| on the left and the corresponding computed respiratory curve $z_{resp}$ (t) on the right.

Fig. 4 shows the isolated cardiac part of the spectrum |$z_{card}$ (f)| on the left and the corresponding computed cardiac curve $z_{card}$ (t) on the right.

Similarly, the heart contraction signal can be determined (see Fig. 4).

Cardiac motion can now be separated by confining the spectrum to cardiac frequencies also taking into account Δf:

$$Z_{card}\left( ||f| - f_{card}| < \Delta f /_2 \right) = Z(f),$$

$$Z_{card}\left( ||f| - f_{card}| > \Delta f /_2 \right) = 0,$$

An inverse Fourier transformation iFFT of Z(f) finally leads to the respiratory curve $z_{card}$ (t):

$$Z_{card}(t) = iFFT[Z_{card}(f)]$$

with which a gating sequence using time-based or amplitude-based gating can easily be established (Fig. 4).

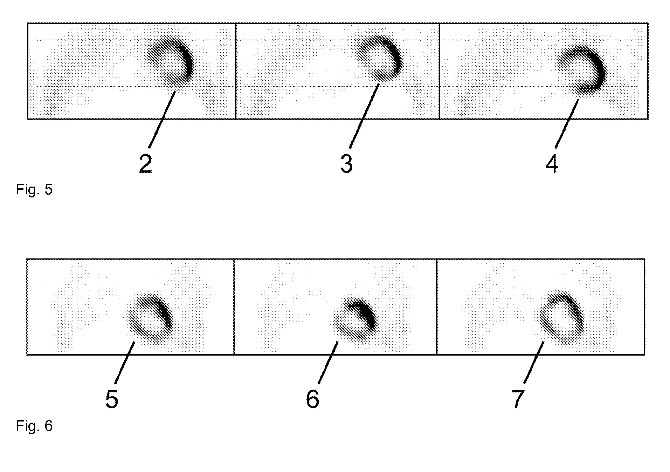

Fig. 5 shows a list mode based respiratory gating (a 20 minute FDG PET scan, 8 respiratory gates). On the left a PET image (2) without gating is shown. In the middle a PET image (3) in maximum expiration is shown and on the right a PET image (4) in maximum inspiration is shown. The respiration induced motion of the left ventricle is clearly visible.

Fig. 6 shows a list mode cardiac gating (a 20 minute FDG PET scan, 10 cardiac gates). On the left a PET image (5) without gating is shown. In the middle a PET image (6) in end systole is shown and on the right a PET image (7) in end diastole is shown. The contraction of the heart is well resolved.

According to another embodiment of the present invention, in cases where the heart contraction is not connected to an axial motion shift, there is no heart beat peak visible in the spectrum. In these cases, a computation not of the distribution's axial center of mass, but of the distribution's standard deviation will reveal a signal of the heart beat which can then be plotted against time as shown in Fig. 2 on the left hand side. As explained above with respect to the preferred embodiment of the present invention, the data is then transformed into the frequency domain allowing isolation of the respiratory and cardiac part of the spectrum as shown in Fig. 3 and Fig. 4 respectively. Using an inverse Fourier transform, respiratory and cardiac curves can then be computed with which a gating sequence (see Fig. 5 and Fig.6) can subsequently be established.

According to yet another embodiment of the present invention, after having divided the data stream (of PET coincidence data) into time frames of a given length, having computed a histogram A(i, t) of an axial coincidence distribution for a set of time frames and having computed the axial center of mass z(t) for each of the time frames in the set of time frames based on the histogram A(i, t), as (see Fig. 2 left hand side) instead of using a Fourier analysis in order to isolate certain parts of the spectrum, a Savitzky Golay filter can be applied to the raw curve z(t), effectively suppressing higher frequencies and resulting in a pure respiratory signal. From the respiratory curve obtained, a gating sequence can in turn be established.

An amplitude-driven gating instead of a time-based scheme is known to have the best ability to resolve the respiratory motion; this scheme accounts for different breathing patterns. For heart contraction, a time-based scheme is usually sufficient; here, the time interval between two signal maxima is divided into equidistant gates. This gating scheme is of advantage in the proposed invention, as the heart signal features beat waves, making amplitude information not easy to obtain; however, time information is well preserved.

The described gating method was verified in a patient study comprising 14 patients who underwent an ECG-gated myocardial viability FDG scan on a Siemens Biograph Sensation 16 PET/CT scanner in List mode. The obtained gated images were compared to gated images derived using a gating based on a video camera monitoring a marker placed on the patient's abdomen as well as the non-gated PET image. The study demonstrated a significantly superior respiratory motion resolution when using the list mode-based method. This was verified by measuring both the maximum observable motion of the left ventricle and ventricular wall thicknesses. These results clearly show that internal heart motion information is superior to motion data derived by monitoring external markers.

The proposed cardiac gating was compared to an ECG-based gating. In average, the measured ejection fractions (defined as the difference of end-diastolic and end-systolic left ventricular volume, divided by the end-diastolic volume) were slightly smaller than the measured ECG-based ejection fractions. However, in cases where there was an overall high uptake in the myocardium, both values were similar, and the heart contraction cycle was well resolved (Fig. 6). Therefore, list mode-based cardiac gating may be used for additional reduction of motion in the PET data sets.

The method according to the present invention therefore allows extracting internal motion information of the heart directly from the PET data itself. This is done without additional hardware. Additionally, it is very time-efficient and superior to respiratory gating methods that rely on external motion information.

**Claims**

1. Method for extracting internal organ motion from positron emission tomography (PET) coincidence data, the method comprising the following steps:

   generating a data stream of PET coincidence data using the list mode capability of a PET scanner;
   dividing the data stream into time frames of a given length;
   computing a histogram A(i, t) of the axial coincidence distribution for a set of time frames;
   computing the axial center of mass z(t) for each of the time frames in the set of time frames based on the histogram A(i, t);
   transforming z(t) into the frequency domain;
   determining either the frequency contribution caused by respiratory motion, given by $f_{resp}$, or the frequency contribution caused by heart contractions, given by $f_{card}$ and $\Delta f$, identified in the frequency spectrum $|Z(f)|$;
   carrying out further processing of Z(f) leading to curves $z_{resp}(t)$ and $z_{card}(t)$ with which a gating sequence is established.

2. Method for extracting internal organ motion from positron emission tomography (PET) coincidence data, the method comprising the following steps:

   generating a data stream of PET coincidence data using the list mode capability of a PET scanner;
   dividing the data stream into time frames of a given length;
   computing a histogram A(i, t) of the axial coincidence distribution for a set of time frames;
   computing the axial center of mass z(t) for each of the time frames in the set of time frames based on the histogram A(i, t);
   applying a Savitzky Golay filter to the raw curve z(t) leading to a respiratory signal $z_{resp}(t)$ with which a gating sequence is established.

3. Method for extracting internal organ motion from positron emission tomography (PET) coincidence data, the method comprising the following steps:

   generating a data stream of PET coincidence data using the list mode capability of a PET scanner;

dividing the data stream into time frames of a given length;
computing a histogram $A(i, t)$ of the axial coincidence distribution for a set of time frames;
computing the distribution's standard deviation $\Delta z(t)$ based on the histogram $A(i, t)$;
transforming $\Delta z(t)$ into the frequency domain;
determining either the frequency contribution caused by respiratory motion, given by $f_{resp}$, or the frequency contribution caused by heart contractions, given by $f_{card}$ and $\Delta f$, identified in the frequency spectrum $|\Delta Z(f)|$;
carrying out further processing of $\Delta Z(f)$ leading to curves $\Delta z_{resp}(t)$ and $\Delta z_{card}(t)$ with which a gating sequence is established.

4. Method according to one of claims 1 to 3, wherein the list mode data stream comprises coordinates of measured PET coincidences.

5. Method according to one of claims 1 to 3, wherein the further processing of $Z(f)$ comprises carrying out an inverse Fourier transformation (iFFT).

6. Method according to one of claims 1 to 3, wherein $Z(f)$ can represent the spectrum of respiratory frequencies $Z_{resp}$ or the spectrum of heart contraction frequencies $Z_{card}$.

7. Method according to one of claims 1 to 6, wherein $z(t)$ can represent the respiratory curve $z_{resp}(t)$ or the cardiac curve $z_{card}(t)$.

8. Method according to one of claims 1 to 7, wherein the list mode data stream comprises time tags.

9. Method according to one of claims 1 to 7, wherein the length of the time frames can be set to be in a range from 5ms to 200ms.

10. Method according to claims 1 or 2, wherein computing the axial coincidence distribution requires the extraction of the axial coordinate for every coincidence from the list mode data.

11. Method according to claim 10, wherein in case of coincidences belonging to higher segments of the michelogram, a single slice rebinning is performed.

12. Method according to claim 11, wherein with single slice rebinning, prompt and delayed coincidences are taken into account with positive and negative weight, respectively.

13. Method according to one of claims 1 to 12, wherein using a fast fourier transformation (FFT), the axial center of mass $z(t)$ is transformed into the frequency domain.

14. Method according to one of claims 1 to 12, wherein the values for $f_{resp}$, $f_{card}$ and $\Delta f$ are found either manually or, by smoothing the spectrum, automatically.

Figures:

Fig.1

Fig. 2

Fig. 3

Fig. 4

Fig. 5

Fig. 6

Europäisches
Patentamt

European
Patent Office

Office européen
des brevets

**EUROPEAN SEARCH REPORT**

Application Number

EP 08 10 5352

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KLEIN G J ET AL: "Fine-scale motion detection using intrinsic list mode PET information" MATHEMATICAL METHODS IN BIOMEDICAL IMAGE ANALYSIS,, 9 December 2001 (2001-12-09), pages 71-78, XP010584861 ISBN: 978-0-7695-1336-2 * page 71, left-hand column, line 1 - page 77, left-hand column, line 13; figures 1-3 * | 1-10,13, 14 | INV. A61B6/03 |
| D,A | BUNDSCHUH ET AL.: "Postacquisition detection of tumor motion in the lung and upper abdomen using list-mode PET data: a feasibility study" JOURNAL OF NUCLEAR MEDICINE, vol. 48, no. 5, May 2007 (2007-05), pages 758-763, XP002548085 US * page 758, right-hand column, line 1 - page 761, left-hand column, line 14 * | 1,2,4, 8-10 | |
| A | WO 2005/020801 A (LUDWIG INST OF CANCER RES [US]) 10 March 2005 (2005-03-10) * paragraph [0020] - paragraph [0048]; figures 1-6 * | 1-3 | TECHNICAL FIELDS SEARCHED (IPC) A61B G06T G01T |
| A | WO 2007/100955 A (KONINKL PHILIPS ELECTRONICS NV [NL]; PHILIPS CORP [US]; BUSCH MARC [DE]) 7 September 2007 (2007-09-07) * page 3, line 5 - page 11, line 18 * * figures * | 1-3 | |
| A | EP 1 077 383 A (MARCONI MEDICAL SYS INC [US] KONINKL PHILIPS ELECTRONICS NV [NL]) 21 February 2001 (2001-02-21) * paragraph [0029] - paragraph [0031] * | 4,8,10 | |

-/--

The present search report has been drawn up for all claims

| Place of search | Date of completion of the search | Examiner |
|---|---|---|
| The Hague | 14 October 2009 | Chen, Amy |

EPO FORM 1503 03.82 (P04C01)

Europäisches
Patentamt

European
Patent Office

Office européen
des brevets

**EUROPEAN SEARCH REPORT**

Application Number

EP 08 10 5352

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MICHEL DEFRISE ET AL: "Exact and Approximate Rebinning Algorithms for 3-D PET Data" IEEE TRANSACTIONS ON MEDICAL IMAGING, IEEE SERVICE CENTER, PISCATAWAY, NJ, US, vol. 16, no. 2, 1 April 1997 (1997-04-01), XP011035619 ISSN: 0278-0062 * abstract * * page 145, right-hand column, line 40 - page 147, right-hand column, line 15 * * figures 1,2,5 * | 11,12 | |
| A | DEFRISE M ET AL: "Continuous and Discrete Data Rebinning in Time-of-Flight PET" IEEE TRANSACTIONS ON MEDICAL IMAGING, IEEE SERVICE CENTER, PISCATAWAY, NJ, US, vol. 27, no. 9, 1 September 2008 (2008-09-01), pages 1310-1322, XP011226131 ISSN: 0278-0062 * abstract * * page 1316, left-hand column, line 1 - page 1320, left-hand column, line 7 * | 11,12 | TECHNICAL FIELDS SEARCHED (IPC) |
| A | US 2006/178575 A1 (PIACSEK KELLY L [US] ET AL PIACSEK KELLY LYNN [US] ET AL) 10 August 2006 (2006-08-10) * paragraph [0002] - paragraph [0003] * * paragraph [0034] - paragraph [0037] * * paragraph [0051] * * figures 1-3 * | 1 | |

The present search report has been drawn up for all claims

| Place of search | Date of completion of the search | Examiner |
|---|---|---|
| The Hague | 14 October 2009 | Chen, Amy |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   ..............................................................................

& : member of the same patent family, corresponding
   document

EPO FORM 1503 03.82 (P04C01)

## EP 2 163 201 A1

ANNEX TO THE EUROPEAN SEARCH REPORT
ON EUROPEAN PATENT APPLICATION NO.

EP 08 10 5352

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2009

| Patent document cited in search report | | Publication date | Patent family member(s) | | Publication date |
|---|---|---|---|---|---|
| WO 2005020801 | A | 10-03-2005 | EP | 1665125 A2 | 07-06-2006 |
| WO 2007100955 | A | 07-09-2007 | CN | 101454801 A | 10-06-2009 |
| | | | EP | 1991959 A2 | 19-11-2008 |
| | | | JP | 2009528139 T | 06-08-2009 |
| EP 1077383 | A | 21-02-2001 | AT | 384272 T | 15-02-2008 |
| | | | DE | 60037779 T2 | 08-01-2009 |
| | | | JP | 2001356172 A | 26-12-2001 |
| | | | US | 6294788 B1 | 25-09-2001 |
| US 2006178575 | A1 | 10-08-2006 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Lang N ; Dawood M ; Büther F ; Schober O ; Schäfers M ; Schäfers K.** Organ Movement-Reduction in PET/CT using Dual-Gated List mode Acquisition. *Z Med Phys.,* 2006, vol. 16, 93-100
- **Souvatzaglou M ; Bengel F ; Busch R et al.** Attenuation correction in cardiac PET/CT with three different CT protocols: a comparison with conventional PET. *Eur J Nucl Med Mol Imaging,* 2007, vol. 34, 1991-2000
- **Klein GJ ; Reutter BW ; Ho MW ; Reed JH ; Huesman RH.** Real-time system for respiratory-cardiac gating in positron tomography. *IEEE Trans Nucl Sci.,* 1998, vol. 45, 2139-2143
- **Nehmeh SA ; Erdi YE ; Ling CC et al.** Effects of Respiratory Gating on Reducing Lung Motion Artifacts in PET Imaging of Lung Cancer. *Med Phys.,* 2002, vol. 29 (3), 366-371
- **Dawood M ; Büther F ; Lang N ; Schober O ; Schäfers KP.** Respiratory gating in positron emission tomography: a comparison of different gating schemes. *Med Phys.,* 2007, vol. 34 (7), 3067-3076
- **Boucher L ; Rodrigue S ; Lecomte R ; Bernard F.** Respiratory gating for 3-dimensional PET of the thorax: Feasibility and initial results. *J Nucl Med.,* 2004, vol. 45, 214-2I9
- **Nehmeh SA ; Erdi YE ; Rosenzweig KE.** Reduction of respiratory motion artifacts in PET imaging of lung cancer by respiratory correlated dynamic PET: methodology and comparison with respiratory gated PET. *J Nucl Med.,* 2003, vol. 44, 1644-1648
- **Koch N ; Liu HH ; Starkschall G et al.** Evaluation of internal lung motion for respiratory-gated radiotherapy using MRI: Part I-correlating internal lung motion with skin fiducial motion. *Int J Radiat Oncol Biol Phys.,* 2004, vol. 60 (5), 1459-1472
- **Bundschuh R A ; Martinez-Moeller A ; Essler M et al.** Postacquisition Detection of Tumor Motion in the Lung and Upper Abdomen Using list mode PET Data: A Feasibility Study. *J Nud Med.,* 2007, vol. 48, 758-763